# EUROPEAN PATENT SPECIFICATION

(11) **EP 4 286 827 B1**
(45) Date of publication and mention of the grant of the patent: **14.01.2026**
(21) Application number: 23175453.2
(22) Date of filing: 25.05.2023
(51) Int. Cl.: G01N 15/10, G01N 35/00, G01N 33/493

(54) **QUALITY CONTROL SUBSTANCE USED IN URINE SEDIMENT ANALYZER**
QUALITÄTSKONTROLLSUBSTANZ ZUR VERWENDUNG IN URINSEDIMENTANALYSEGERÄT
SUBSTANCE DE CONTRÔLE DE QUALITÉ UTILISÉE DANS UN ANALYSEUR DE SÉDIMENTS D'URINE

(30) Priority: 31.05.2022 JP 2022088489; 16.05.2023 JP 2023080930
(43) Date of publication of application: 06.12.2023
(73) Proprietor: ARKRAY, Inc., Kyoto-shi, Kyoto 601-8045 (JP)
(72) Inventor: HIROTA, Takuma, Kyoto-shi, 602-0008 (JP); NAKAO, Atsushi, Kyoto-shi, 602-0008 (JP); NAKAJIMA, Shinya, Kyoto-shi, 602-0008 (JP)
(74) Representative: Pitchford, James Edward

(56) References cited:
- WO-A1-2021/222076
- CN-A- 107 153 020
- US-B2- 10 437 036

## Description

### TECHNICAL FIELD

The present disclosure relates to a quality control substance for urine sediment examination, which substance is used for quality control in a urine sediment analysis using a urine sediment analyzer.

### BACKGROUND ART

A urine sediment analyzer is used for evaluating the presence of various analyte species of sediments in a urine sample, based on the forms of the sediments in the urine sample. Quality control is required in order for the urine sediment analyzer to accurately detect and classify the analyte species based on the forms of the sediments.

As a quality control substance intended for use in a conventional quality control, for example, Non-patent Document 1 discloses a control urine for urine sediment examination, obtained by incorporating erythrocytes and leukocytes separated from human-derived blood and immobilized with glutaraldehyde, into a pooled urine.

Further, Patent Document 1 discloses a quality control substance which includes: a cancer cell as a substance that indicates the form of a non-squamous epithelial cell in urine; an algae cell as a substance that indicates the form of an abnormal cast in urine; a yeast cell as a substance that indicates the form of a yeast cell in urine; and egg white as a substance that indicates the form of mucus in urine. In other words, an object of the technique disclosed in Patent Document 1 is to perform quality control, in the case of observing a urine sample with a microscope, by using other substances whose forms are similar to urine sediments in the sample, and comparing the forms of components in the quality control substance with the forms of analytes in the urine sample. More specifically, commercially available human breast cancer cells (SKBR-3 cells) and human lung cancer cells (H-1975 cells) are used in Patent Document 1, as the substances similar to the forms of "non-" squamous epithelial cells.

### PRIOR ART DOCUMENTS

### PATENT DOCUMENTS

[Patent Document 1] JP2020-531852A
[Patent Document 2] JP2019-066461A

### NON-PATENT DOCUMENTS

[Non-patent Document 1] Nobuko Imai et al., Hygiene Examination, Vol. 39, Issue 1, 1990, p. 55 to 61

Further background art is provided in WO 2021/222076 A1 and CN 107 153 020 A.

WO 2021/222076 A1 discloses a system for detecting the presence of interfering conditions in the results from a testing apparatus, the system comprising: a testing apparatus that measures particles included in a urine sample; and a system processor communicatively coupled with the testing apparatus, the system processor coupled to a memory storing instructions that, when executed by the system processor, cause the system processor to: receive detection results of a urine sample from the testing apparatus, the results including (i) a first detection measurement of a first analyte present in the urine sample and (ii) a second detection measurement of a second analyte present in the urine sample; select a user-configurable interference validation condition; compare the first detection measurement to the selected user-configurable interference validation condition; compare the second detection measurement to the selected user-configurable interference validation condition; generate one or more interfering flags if each of the first detection measurement and the second detection measurement meet the selected user-configurable interference validation condition; store the generated interfering flag in the memory; and display the interfering flag.

CN 107 153 020 A discloses a preparation method of a urinary formed element quality control product.

### SUMMARY

In the urine sediment analysis using a urine sediment analyzer disclosed in Patent Document 1 or Non-patent Document 1, it is possible to perform quality control in the analysis of erythrocytes, leukocytes, non-squamous epithelial cells, abnormal casts, yeast cells, and mucus that can be present in urine, using the quality control substance. However, quality control cannot be performed for sediment items that can be present in urine, other than those mentioned above. For example, no quality control substance for performing quality control in the analysis of squamous epithelial cells that can be present in urine, has been reported at the moment. Further, it has also been desired to obtain a quality control substance with an easily available material(s).

An object of the present disclosure is to provide a quality control substance which is used in a urine sediment analysis using a urine sediment analyzer, and which is for performing quality control in the analysis of squamous epithelial cells that can be present in urine.

The present inventors and others have conducted intensive studies to solve the above-mentioned problems, and as a result, found out that the form of squamous epithelial cells derived from urinary tract, included in the items of squamous epithelial cells in the urine sediment examination, are extremely similar to the form of a specific type of squamous epithelial cells derived from a mammal, and that the specific type of squamous epithelial cells derived from a mammal can be suitably used as a quality control substance to be used in a urine sediment analysis using a urine sediment analyzer.

In general, the forms of cells vary significantly depending on the animal species of origin, the site of origin, the type of the cells, and the like. Therefore, it is thought that even those skilled in the art usually cannot conceive of the fact that squamous epithelial cells derived from urinary tract are similar in form to a specific type of squamous epithelial cells derived from a mammal, particularly, mucosal epithelial cells derived from human oral cavity, or surface mucous cells derived from bovine stomach, cervine stomach, sheep stomach or porcine stomach. Accordingly, it was an unexpected discovery that the above-described specific type of squamous epithelial cells can be used as a quality control substance in a urine sediment analysis using a urine sediment analyzer.

Specifically, the present invention is defined in the appended claims. Details of certain embodiments are set out in the dependent claims.

### EFFECTS

According to the quality control substance according to the present disclosure, it is possible to perform quality control in a preferred manner at the time of analyzing squamous epithelial cells that can be present in urine, particularly, in the case of using a urine sediment analyzer. In particular, it is expected that the present disclosure can provide a highly reliable quality control substance, since the same epithelial cells as squamous epithelial cells present in a urine sample are used in the quality control substance. Further, the squamous epithelial cells to be used in the present disclosure can be any mucosal epithelial cells which can be obtained safely and non-invasively from the surface of the human oral cavity, regardless of whether the cells are commercially available or prepared at the time of use. It is also possible to preferably use surface mucous cells obtained from the surface of the bovine reticulum (or honey-comb stomach; namely, the second compartment of the bovine stomach) or the surface of the bovine omasum (or psalterium; namely, the third compartment of the bovine stomach), which can be obtained easily and at a low cost, as a commercially available product. By using such mucosal epithelial cells obtained from the surface of the human oral cavity or commercially available surface mucous cells derived from the bovine stomach, it is possible to obtain squamous epithelial cells having the same quality in a large quantity.

### BRIEF DESCRIPTION OF THE DRAWINGS

FIG. 1 shows images of non-immobilized squamous epithelial cells derived from the human oral cavity, obtained by a urine sediment analyzer, AUTION EYE^{®} AI-4510. The scale bar shown at the lower left of the figure indicates 10 µm.
FIG. 2 shows images of non-immobilized human squamous epithelial cells in urine, as a comparison, obtained by a urine sediment analyzer, AUTION EYE^{®} AI-4510. The scale bar shown at the lower left of the figure indicates 10 µm.
FIG. 3 shows images of non-immobilized squamous epithelial cells derived from the bovine reticulum, obtained by a urine sediment analyzer, AUTION EYE^{®} AI-4510. The scale bar shown at the lower left of the figure indicates 10 µm.
FIG. 4 shows images of non-immobilized squamous epithelial cells derived from the bovine omasum, obtained by a urine sediment analyzer, AUTION EYE^{®} AI-4510. The scale bar shown at the lower left of the figure indicates 10 µm.
FIG. 5 shows unstained and S-stained microscopic photographs of real urine, non-immobilized squamous epithelial cells derived from the human oral cavity, and non-immobilized squamous epithelial cells derived from the bovine omasum. These photographs are obtained by observation at HPF (400-fold magnification).
FIG. 6 shows images of immobilized squamous epithelial cells derived from the human oral cavity, obtained by a urine sediment analyzer, AUTION EYE^{®} AI-4510. The scale bar shown at the lower left of the figure indicates 10 µm.
FIG. 7 shows images of immobilized squamous epithelial cells derived from the bovine reticulum, obtained by a urine sediment analyzer, AUTION EYE^{®} AI-4510. The scale bar shown at the lower left of the figure indicates 10 µm.
FIG. 8 shows images of immobilized squamous epithelial cells derived from the bovine omasum, obtained by a urine sediment analyzer, AUTION EYE^{®} AI-4510. The scale bar shown at the lower left of the figure indicates 10 µm.
FIG. 9 shows images of immobilized squamous epithelial cells derived from the cervine stomach obtained by a urine sediment analyzer, AUTION EYE^{®} AI-4510. The scale bar shown at the lower left of the figure indicates 10 µm.
FIG. 10 shows images of immobilized squamous epithelial cells derived from the sheep stomach, obtained by a urine sediment analyzer, AUTION EYE^{®} AI-4510. The scale bar shown at the lower left of the figure indicates 10 µm.
FIG. 11 shows images of immobilized squamous epithelial cells derived from the porcine stomach, obtained by a urine sediment analyzer, AUTION EYE^{®} AI-4510. The scale bar shown at the lower left of the figure indicates 10 µm.

### DETAILED DESCRIPTION OF EMBODIMENTS

### < Quality Control Substance >

One embodiment of the present disclosure is a quality control substance including squamous epithelial cells derived from a mammal. The term "mammal" as used herein encompasses (but is not limited to) cattle, humans, deer, sheep, and pigs, for example. The squamous epithelial cells are isolated from the mammal in advance of being used as, or in, the quality control substance. In other words, the squamous epithelial cells are separated and removed from their natural environment within the mammal, for later use as, or in, the quality control substance. It will therefore be appreciated that the use of the squamous epithelial cells, derived from the mammal, as (or in) the quality control substance, takes place "*ex vivo*" or "*in vitro*"*.*

The quality control substance according to the present embodiment is preferably a quality control substance for use in a urine sediment analyzer, and a quality control substance for controlling the detection accuracy for detecting squamous epithelial cells in urine. A commonly used urine sediment analyzer based on image analysis can be used, as the urine sediment analyzer. For example, it is possible to use the urine sediment analyzer disclosed in Patent Document 2, which is based on an unstained image analysis performed in a fluid.

The quality control substance according to the present embodiment may further include an aqueous medium such as water. The aqueous medium is not particularly limited as long as it does not affect the quality control, and may be an aqueous medium having a buffering capacity. As the aqueous medium having a buffering capacity, a common buffer solution which does not affect the quality control, such as phosphate buffered saline, can be preferably used, and a human-derived pooled urine can also be preferably used. In cases where the quality control substance according to the present embodiment contains an aqueous medium having a buffering capacity, the quality control substance preferably has a pH suitable for maintaining the squamous epithelial cells contained therein. The quality control substance may have, for example, a pH of from 7.2 to 7.6, but not particularly limited thereto as long as the quality control substance can be used for the quality control. The squamous epithelial cells derived from a mammal contained in the quality control substance preferably have a concentration in the aqueous medium of from 0.1 to 600 cells/µl, but not particularly limited thereto as long as the quality control substance can be used for the quality control.

The squamous epithelial cells to be included in the quality control substance according to the present embodiment may be non-immobilized or immobilized.

The quality control substance may be prepared one by one, by adding squamous epithelial cells, preferably to the aqueous medium having a buffering capacity, immediately before performing the quality control. It is also possible to immobilize the squamous epithelial cells and to mix with the aqueous medium having a buffering capacity, in advance, so that the preservation stability can be increased by the immobilization. Further, the immobilization serves to homogenize the specific gravity, and the surface condition of the cells, to reduce variation between the cells. This increases the possibility that the layer of flowing squamous epithelial cells, when flowing through a flow cell in a urine sediment analysis, is constant. As a result, it becomes easier to focus, at the time of calibration in an optical measurement.

The squamous epithelial cells are immobilized in a state where the non-spherical shape of the cells is maintained.

The squamous epithelial cells can be immobilized by any method as long as the method allows for maintaining the non-spherical shape of the squamous epithelial cells. For example, the cells can be immobilized by a known immobilization technique, using an aldehyde such as glutaraldehyde or formaldehyde. The squamous epithelial cells can be immobilized, for example, by allowing the cells to stand at room temperature for 60 minutes in a 1% glutaraldehyde solution, but not particularly limited thereto as long as the cells can be immobilized.

The mammal from which the squamous epithelial cells to be included in the quality control substance according to the present embodiment are derived, is not particularly limited, as long as the squamous epithelial cells derived therefrom are similar in form to the squamous epithelial cells present in human urine, and can be used as a quality control substance in a urine sediment analysis using a urine sediment analyzer. For example, the mammal may be one or more mammals selected from the group consisting of cattle, humans, deer, sheep, and pigs. In other words, the quality control substance according to the present embodiment may include squamous epithelial cells derived from only one mammal or a plurality of mammals included in the above-mentioned group.

Specifically, the squamous epithelial cells to be included in the quality control substance according to the present embodiment may be mucosal epithelial cells derived from human oral cavity, and may be surface mucous cells derived from bovine stomach, cervine stomach, sheep stomach, or porcine stomach. The squamous epithelial cells to be included in the quality control substance according to the present embodiment are preferably mucosal epithelial cells derived from the human oral cavity, or surface mucous cells derived from the bovine stomach, since these cells have a particularly high similarity in form to the squamous epithelial cells present in human urine. The bovine stomach as used herein is preferably bovine reticulum or bovine omasum.

The squamous epithelial cells to be included in the quality control substance according to the present embodiment are preferably cells which have a non-spherical flattened shape, and whose form is amorphous and asymmetric, with thin cytoplasm and twisted or bent margins. The squamous epithelial cells preferably have such a size that the longer diameter thereof is from about 20 to 100 µm, and particularly preferably have a size (with a longer diameter of from 30 to 70 µm) that is about the same as the size of human squamous epithelial cells in urine.

The squamous epithelial cells to be included in the quality control substance according to the present embodiment may be collected from any of various mammals, by a method known to those skilled in the art, or may be collected from a commercially available stomach of any of various mammals. Further, any of various types of squamous epithelial cells may be purchased as a commercially available product(s), or may be cultured and used, as necessary. All of these types of squamous epithelial cells have an advantage of being easily available.

For example, squamous epithelial cells derived from the human oral cavity can be collected by swabbing the inner surface of both cheeks in the oral cavity of a human about 10 to 20 times, using a cell collecting tool such as a cotton swab, a spatula, or a brush. The squamous epithelial cells adhered to the cell collecting tool may be suspended in a solvent such as PBS or physiological saline, and washed once or multiple times by centrifugation or the like.

Further, surface mucous cells of the stomach of any of various mammals can be collected, for example, by swabbing the surface mucous cells present on the surface of the stomach of any of various mammals about 10 to 20 times, using a cell collecting tool such as a cotton swab, a spatula or a brush. By suspending the squamous epithelial cells adhered to the cell collecting tool in a solvent such as PBS or physiological saline, and filtering the resulting suspension, it is possible to separate a fraction of cells having a size (with a longer diameter of from 30 to 70 µm) that is about the same as the size of human squamous epithelial cells in urine. The separated cells may further be washed once or multiple times by centrifugation or the like.

In addition, as the squamous epithelial cells of any of various mammals, for example, the cells that had been cultured as necessary, according to culture conditions suitable for culturing each type of squamous epithelial cells, may be used.

The quality control substance according to the present embodiment may further include one or more quality control substances that correspond to measurement items to be measured in the urine sediment examination, other than squamous epithelial cells, for example, measurement items such as erythrocytes, leukocytes, leukocyte agglomerates, non-squamous epithelial cells, hyaline casts, other casts, bacteria (bacilli), crystals, yeast cells, mucus threads, sperms and the like. This allows for simultaneously performing the quality control for the respective measurement items in the urine sediment examination. Further, each of these quality control substances may be optionally immobilized.

The quality control substance according to the present embodiment may further include an additional component(s). Examples of the additional component include pH adjusting agents (such as HCl and NaCl), osmotic pressure regulators (such as salts), components to be measured with a test paper for a urine qualitative test (such as glucose, proteins (albumin and the like), bilirubin, urobilinogen, hemoglobin, ketone bodies, nitrous acid, and enzymes such as elastase), main components constituting urine matrices (such as salts (NaCl, KCl, and the like), organic acids (such as oxalic acid), proteins (such as albumin and Tamm-Horsfall Protein), uric acid, urea, and phosphoric acid. However, it is better not to incorporate components that cause clouding, such as a large amount of bacteria.

### < Method for Detecting the Presence of Squamous Epithelial Cells in Urine sample >

Another embodiment of the present disclosure is a method for detecting the presence of squamous epithelial cells in a urine sample derived from a subject, the method including the steps of:
analyzing the quality control substance described in the section of < Quality Control Substance > described above, using a urine sediment analyzer, in order to determine the form of squamous epithelial cells in the quality control substance;
analyzing (*ex vivo* or *in vitro*) the urine sample derived from the subject, using the urine sediment analyzer;
comparing the form of the squamous epithelial cells in the quality control substance with the form of an analyte in the urine sample; and
determining that squamous epithelial cells are present in the urine sample, if the form of the analyte in the urine sample matches the form of the squamous epithelial cells in the quality control substance.

The "subject" refers to an individual who undergoes the urine sediment examination. The subject may be an individual with a disease, an individual suspected to have a disease, or a healthy individual. The subject is preferably a human being.

Whether or not the form of the analyte in the urine sample matches the form of the squamous epithelial cells in the quality control substance can be automatically determined by the urine sediment analyzer to be used.

For example, in the case of using the urine sediment analyzer disclosed in Patent Document 2, the analyzer that determines the type of a sediment component in a urine sample, extracts feature values (such as color, form, size, and the like) from the images of the sediment component; also extracts feature values from the images of the quality control substance; determines whether or not the forms of both match with each other, by the pattern matching of the respective feature values; and indicates that squamous epithelial cells are present in the urine sample when it is determined that the forms of both match with each other.

Further, the urine sediment analyzer may count the number of the squamous epithelial cells detected.

The definition of the urine sediment analyzer includes a urine sediment analyzer based on a microscope. The urine sediment analyzer based on a microscope may be, for example, AUTION EYE^{®} AI-4510 (manufactured by Arkray, Inc.).

By detecting the presence of squamous epithelial cells in a urine sample derived from a subject, or by the result of counting the number of the squamous epithelial cells in the urine sample derived from the subject, it is possible to suspect the presence of a urinary tract disease, such as urinary tract infection or urolithiasis in the subject, and to determine a treatment plan (for example, the administration of an anti-microbial agent, an anti-viral agent, or any other drugs) for such a disease, as necessary.

### < Method for Controlling Measurement Accuracy >

Another embodiment of the present disclosure is a method for controlling the measurement accuracy of an analyzer that measures the cell number concentration of squamous epithelial cells in a sample, by counting the number of particles whose form is similar to the form of the squamous epithelial cells in the sample, the method including the steps of:
measuring the cell number concentration of squamous epithelial cells in the quality control substance described in the section of < Quality Control Substance >, using the analyzer, to obtain a measured value; and
determining the measurement accuracy of the analyzer by comparing the measured value with a reference concentration.

The "cell number concentration" refers to the number of cells contained in a unit volume, which is also referred to as "number concentration".

The analyzer is, for example, an analyzer including a microscope, and a camera for imaging the particles in the sample. The analyzer acquires an image in which the particles in the sample are captured, by imaging the particles in the sample with the camera via the microscope; counts the number of particles (similar particles) which are captured in the acquired image and whose degree of similarity with the form of the squamous epithelial cells stored in the analyzer in advance is equal to or higher than a predetermined value, as squamous epithelial cells; and measures the particle number concentration of the similar particles in the sample as the cell number concentration of the squamous epithelial cells, based on the counted value. The analyzer that measures the cell number concentration of squamous epithelial cells in a sample may be, for example, a urine sediment analyzer based on a microscope, and the urine sediment analyzer based on a microscope may be, for example, AUTION EYE^{®} AI-4510 (manufactured by Arkray, Inc.).

The sample is not particularly limited as long as the sample possibly contains leukocytes. Examples of the sample include urine and blood. Of these, urine is preferred.

When the quality control substance is analyzed using the analyzer, the squamous epithelial cells included in the quality control substance are counted as similar particles, and the measured value of the cell number concentration of the squamous epithelial cells included in the quality control substance is obtained. The measurement accuracy of the analyzer may be determined by comparing the measured value with a reference concentration, and based, for example, on the difference between the measured value and the reference concentration. In this case, the analyzer can be determined to have a good measurement accuracy, if the difference is less than a predetermined value. In contrast, the analyzer can be determined to have a poor measurement accuracy, if the difference is equal to or higher than the predetermined value.

Further, the measurement accuracy of the analyzer may also be determined based on the proportion of the measured value with respect to the reference concentration. In this case, the analyzer can be determined to have a good measurement accuracy, if the proportion is less than a predetermined value. In contrast, the analyzer can be determined to have a poor measurement accuracy, if the proportion is equal to or higher than the predetermined value. The predetermined value can be set as appropriate depending on the accuracy required for the analyzer.

The reference concentration (also referred to as "reference cell number concentration) can be, for example: the value obtained by analyzing the same quality control substance with a plurality of analyzers to obtain a plurality of measured values of the particle number concentration of similar particles (squamous epithelial cells), and averaging the thus obtained measured values; the measured value of the particle number concentration of similar particles obtained by measuring the quality control substance by microscopy; the measured value of the particle number concentration of similar particles obtained by measuring the quality control substance with a quality-controlled analyzer; or the like. The reference concentration can also be the mean value of the particle number concentrations of similar particles, which have been calculated by analyzing the quality control substance multiple times, in advance, with the above-described analyzer.

The accuracy of the microscopy can be controlled by obtaining the measured value of the cell number concentration of the squamous epithelial cells in the quality control substance by microscopy, and comparing the resulting measured value with the reference concentration.

### EXAMPLES

Examples are described for the purpose of disclosure, and are not intended to limit the scope of the present disclosure.

### < Example 1: Analysis of Non-immobilized Squamous Epithelial Cells Derived from Human Oral Cavity by Urine Sediment Analyzer >

Mucosal epithelial cells derived from the human oral cavity were separated from oral tissue by the following procedures (1) to (5), in order to obtain squamous epithelial cells derived from the human oral cavity.
(1) PBS having the following composition was prepared.
   · Sodium chloride: 137 mM
   · Disodium hydrogen phosphate: 10 mM
   · Potassium chloride: 2.7 mM
   · Potassium dihydrogen phosphate: 1.8 mM
(2) The inner surface of both cheeks in the oral cavity of a healthy human was swabbed with a cotton swab about 15 times.
(3) The cotton swab was dipped in the PBS prepared in the above (1), to suspend the mucosal epithelial cells attached to the cotton swab.
(4) The resulting suspension was centrifuged (at 500 G for five minutes), the supernatant was removed, and the PBS prepared in the above (1) was added thereto. The washing operation described above was repeated twice, to remove oral debris and bacteria which had been mixed into the collected mucosal epithelial cells.
(5) The cells were centrifuged (at 500 G for five minutes), the supernatant was removed, and 5 mL of the PBS prepared in the above (1) was added thereto. The washing operation described above was repeated twice.

When the mucosal epithelial cells derived from the human oral cavity which had been obtained by the above-described procedures (1) to (5) were analyzed by a urine sediment analyzer, AUTION EYE^{®} AI-4510 manufactured by Arkray, Inc., the cells were detected as squamous epithelial cells (SQEC).

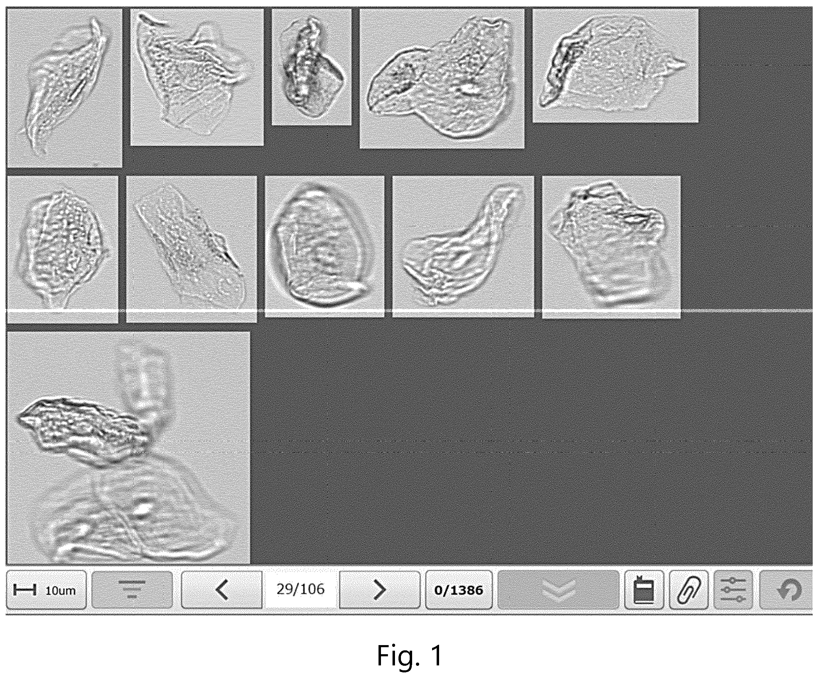

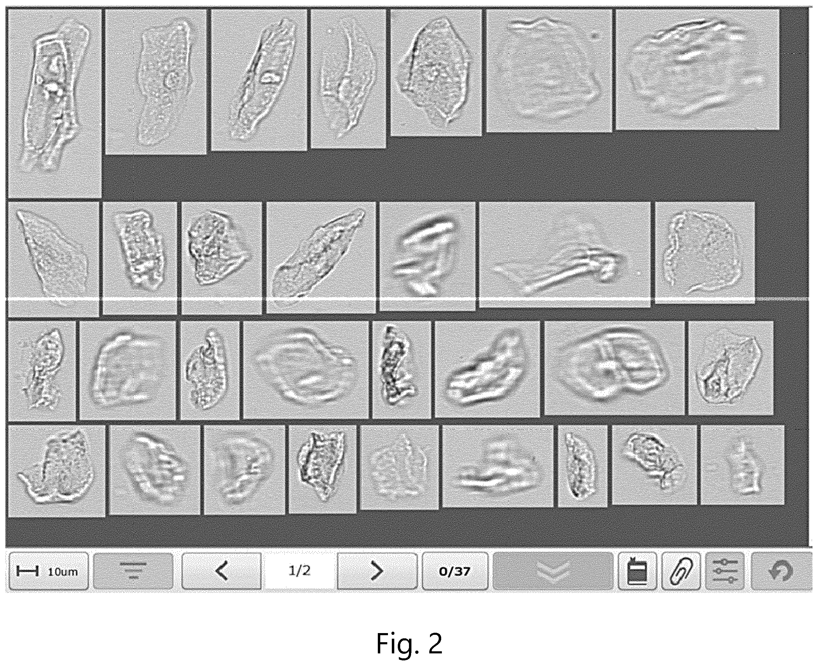

The images of the mucosal epithelial cells derived from the human oral cavity, and the images of non-immobilized human squamous epithelial cells in urine, as a comparison, obtained by AUTION EYE^{®} AI-4510, are shown in FIG. 1 and FIG. 2, respectively. It has been found out that the forms of the cells in both figures are extremely similar to each other, even by visual comparison.

### < Example 2: Analysis of Non-immobilized Squamous Epithelial Cells Derived from Bovine Stomach by Urine Sediment Analyzer >

Surface mucous cells derived from the bovine stomach were separated by the following procedures (1) to (3), in order to obtain squamous epithelial cells derived from the bovine stomach.
(1) A commercially available bovine stomach was purchased, in order to use surface mucous cells located on the surfaces of the bovine reticulum (or honey-comb stomach; namely, the second compartment of the bovine stomach) and the bovine omasum (or psalterium; namely, the third compartment of the bovine stomach).
(2) The surfaces of the above-described reticulum and omasum were each swabbed with a cotton swab to collect surface mucous cells, and each cotton swab was dipped in a physiological saline to suspend the collected cells therein.
(3) Each resulting suspension was filtered, to separate a fraction of cells having a size (with a longer diameter of from 30 to 70 µm) that is about the same as the size of human squamous epithelial cells (SQEC) in urine. The cells were centrifuged (at 500 G for five minutes), the supernatant was removed, and the PBS which is the same as that to be prepared in (1) in Example 4 was added thereto. The washing operation described above was repeated twice.

When the surface mucous cells derived from both bovine stomachs which had been obtained by the above-described procedures (1) to (3) were analyzed by a urine sediment analyzer, AUTION EYE^{®} AI-4510 manufactured by Arkray, Inc., the cells were detected as SQEC.

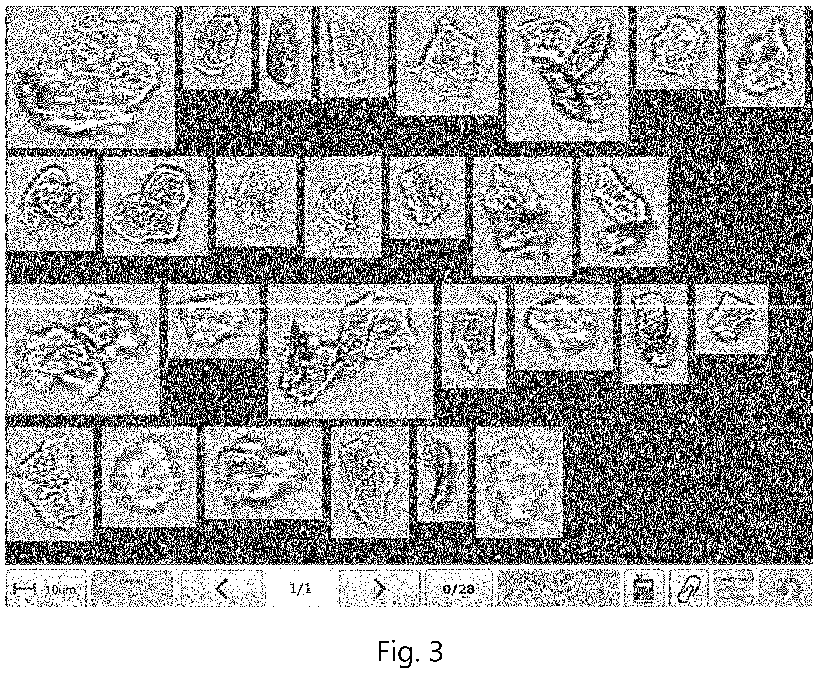

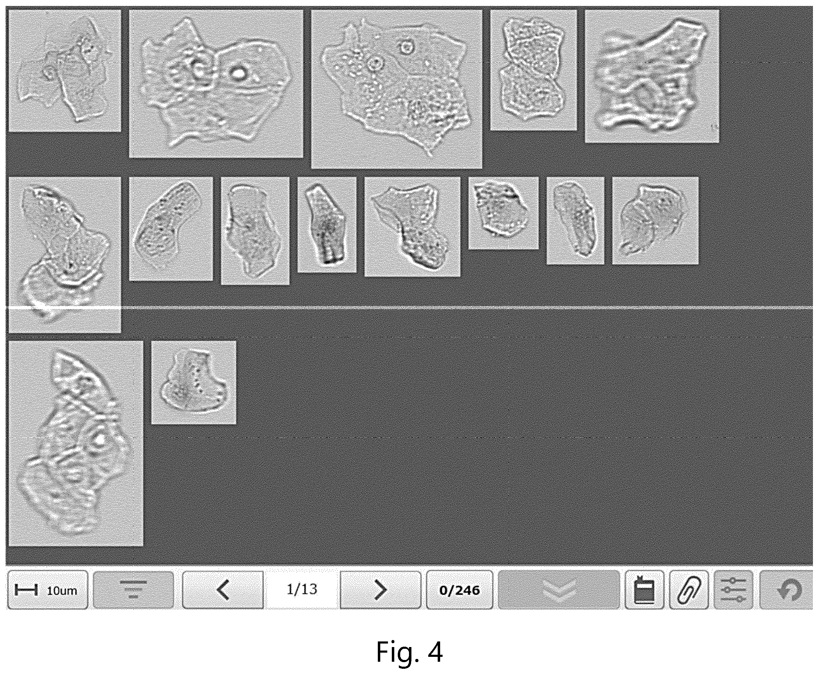

The images of the surface mucous cells derived from the bovine reticulum, and the images of the surface mucous cells derived from the bovine omasum, obtained by AUTION EYE^{®} AI-4510, are shown in FIG. 3 and FIG. 4, respectively. It has been found out that the forms of the cells in both figures are extremely similar to the form of cells in FIG. 2 (the images of non-immobilized human squamous epithelial cells in urine), even by visual comparison.

### < Example 3: Analysis of Real Urine, Non-immobilized Squamous Epithelial Cells derived from Human Oral Cavity, and Non-immobilized Squamous Epithelial Cells derived from Bovine Omasum, by Microscopic Observation >

Real urine, squamous epithelial cells derived from the human oral cavity, and squamous epithelial cells derived from the bovine omasum were observed by microscopy. The samples used are as follows.
· Real urine (urine of a patient obtained from a hospital)
· Oral epithelial cells (cells of a healthy individual collected in the research laboratory of Arkray, Inc.)
· Bovine omasum epithelial cells (cells obtained by the method described in Example 2)

Each sample was treated by the following procedures (1) to (6).
(1) Each sample was prepared. The real urine was used as it is. The oral epithelial cells and the bovine omasum epithelial cells were each suspended in the PBS which is the same as that prepared in (1) in Example 1.
(2) Ten mL of each sample was dispensed in a urine sediment spitz tube.
(3) Each sample was centrifuged at 1,500 rpm and 25°C, for five minutes.
(4) The supernatant was discarded, and 200 µL of the residue was saved.
(5) Fifteen µL of the residue of each sample was collected, and observed with a microscope.
(6) The remaining residue of each sample was subjected to S staining by a method known to those skilled in the art, using LABOSTAIN S (No. 50181, manufactured by Muto Pure Chemicals Co., Ltd.), and observed with a microscope again.

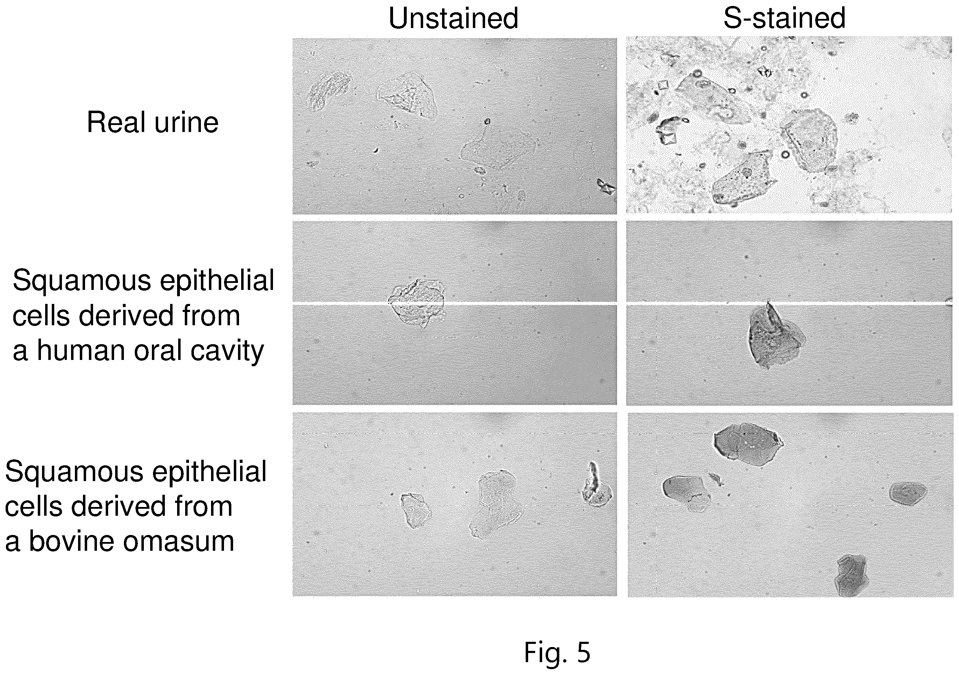

The results are shown in FIG. 5. In the real urine, squamous epithelial cells commonly observed in urine were observed. The squamous epithelial cells had an amorphous shape, with thin cytoplasm and twisted or bent margins. The squamous epithelial cells showed a good stainability with S staining, and were stained reddish violet. The oral epithelial cells and the bovine omasum epithelial cells had an amorphous shape, with thin cytoplasm and bent margins, as with the squamous epithelial cells in urine. Both types of these epithelial cells showed a good cytoplasmic and nuclear stainability with S staining, and were stained reddish violet.

### < Example 4: Analysis of Immobilized Squamous Epithelial Cells Derived from Human Oral Cavity by Urine Sediment Analyzer >

Mucosal epithelial cells derived from the human oral cavity were immobilized by the following procedures (1) to (7), to obtain immobilized squamous epithelial cells.
(1) PBS having the following composition was prepared.
   · Sodium chloride: 137 mM
   · Disodium hydrogen phosphate: 10 mM
   · Potassium chloride: 2.7 mM
   · Potassium dihydrogen phosphate: 1.8 mM
(2) The inner surface of both cheeks in the oral cavity of a healthy human was swabbed with a cotton swab about 15 times.
(3) The cotton swab was dipped in the PBS prepared in the above (1), to suspend the mucosal epithelial cells attached to the cotton swab.
(4) The resulting suspension was centrifuged (at 500 G for five minutes), the supernatant was removed, and the PBS prepared in the above (1) was added thereto. The washing operation described above was repeated twice, to remove oral debris and bacteria which had been mixed into the collected mucosal epithelial cells.
(5) Glutaraldehyde was added thereto so as to achieve a concentration of 1%, followed by mixing by inversion.
(6) The cells were immobilized by allowing to stand for 60 minutes.
(7) The cells were centrifuged (at 500 G for five minutes), the supernatant was removed, and 5 mL of the PBS prepared in the above (1) was added thereto. The washing operation described above was repeated twice.

When the immobilized squamous epithelial cells derived from the human oral cavity which had been obtained by the above-described procedures (1) to (7) were analyzed by a urine sediment analyzer, AUTION EYE^{®} AI-4510 manufactured by Arkray, Inc., the cells were detected as squamous epithelial cells (SQEC).

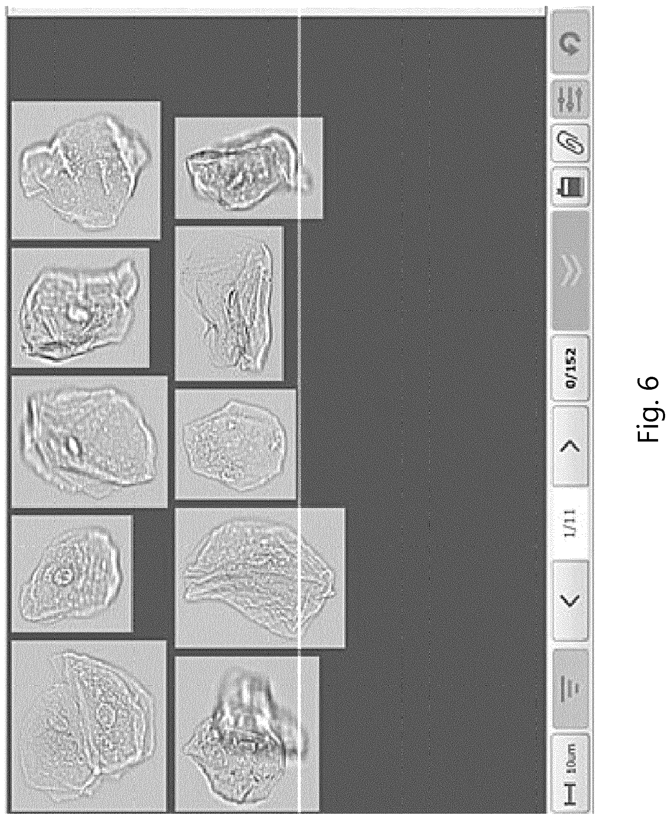

The images of the immobilized squamous epithelial cells derived from the human oral cavity, and the images of non-immobilized human squamous epithelial cells in urine, as a comparison, obtained by AUTION EYE^{®} AI-4510, are shown in FIG. 6 and FIG. 2, respectively. It has been found out that the forms of the cells in both figures are extremely similar to each other, even by visual comparison.

### < Example 5: Analysis of Immobilized Squamous Epithelial Cells Derived from Bovine Stomach by Urine Sediment Analyzer >

Surface mucous cells derived from the bovine stomach were immobilized by the following procedures (1) to (6), to obtain immobilized squamous epithelial cells.
(1) A commercially available bovine stomach was purchased, in order to use surface mucous cells located on the surfaces of the bovine reticulum (or honey-comb stomach; namely, the second compartment of the bovine stomach) and the bovine omasum (or psalterium; namely, the third compartment of the bovine stomach).
(2) The surfaces of the above-described reticulum and omasum were each swabbed with a cotton swab to collect surface mucous cells, and each cotton swab was dipped in a physiological saline to suspend the collected cells therein.
(3) Each resulting suspension was filtered, to separate a fraction of cells having a size (with a longer diameter of from 30 to 70 µm) that is about the same as the size of human squamous epithelial cells (SQEC) in urine.
(4) Glutaraldehyde was added thereto so as to achieve a concentration of 1%, followed by mixing by inversion.
(5) The cells were immobilized by allowing to stand for 60 minutes.
(6) The cells were centrifuged (at 500 G for five minutes), the supernatant was removed, and the PBS which is the same as that prepared in (1) in Example 1 was added thereto. The washing operation described above was repeated twice.

When the immobilized squamous epithelial cells derived from both bovine stomachs which had been obtained by the above-described procedures (1) to (6) were analyzed by a urine sediment analyzer, AUTION EYE^{®} AI-4510 manufactured by Arkray, Inc., the cells were detected as SQEC.

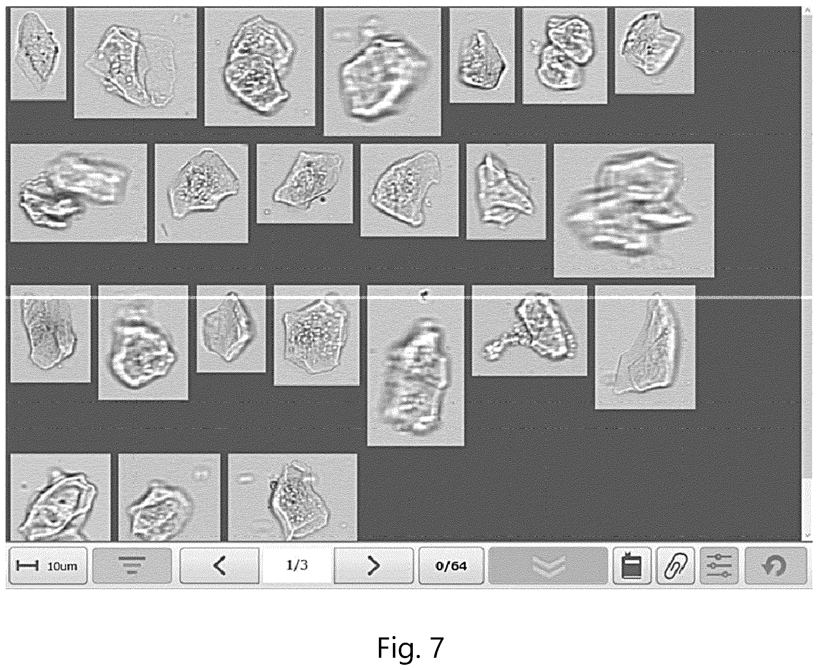

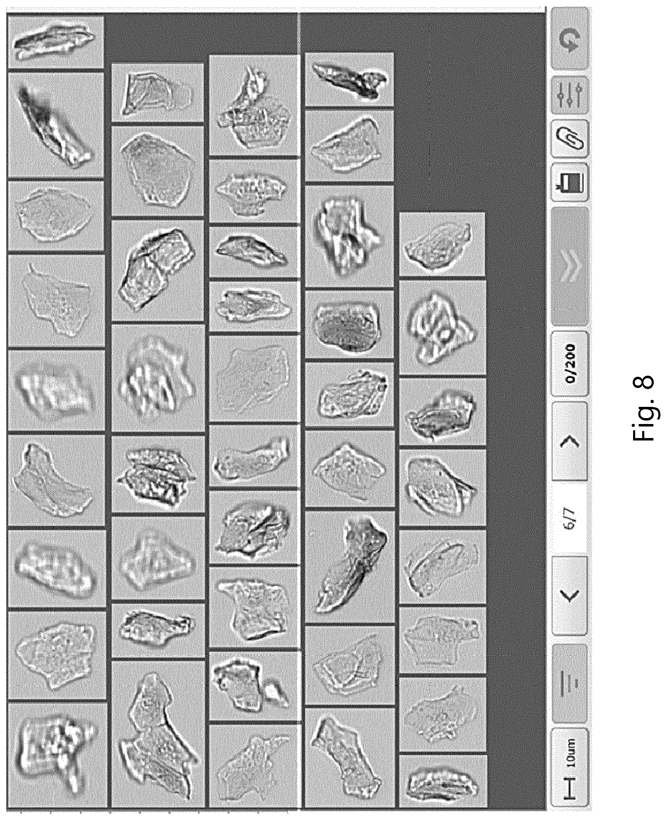

The images of the immobilized squamous epithelial cells derived from the bovine reticulum, and the images of the immobilized squamous epithelial cells derived from the bovine omasum, obtained by AUTION EYE^{®} AI-4510, are shown in FIG. 7 and FIG. 8, respectively. It has been found out that the forms of the cells in both figures are extremely similar to the form of cells in FIG. 2 (the images of non-immobilized human squamous epithelial cells in urine), even by visual comparison.

### < Example 6: Analysis of Immobilized Squamous Epithelial Cells Derived from Cervine Stomach, Sheep Stomach, and Porcine Stomach by Urine Sediment Analyzer >

Next, surface mucous cells derived from each of the cervine, sheep, and porcine stomachs were immobilized, to obtain immobilized squamous epithelial cells. The immobilized squamous epithelial cells were obtained by the same procedure as that used for obtaining the immobilized squamous epithelial cells derived from both bovine stomachs which are described in Example 5.

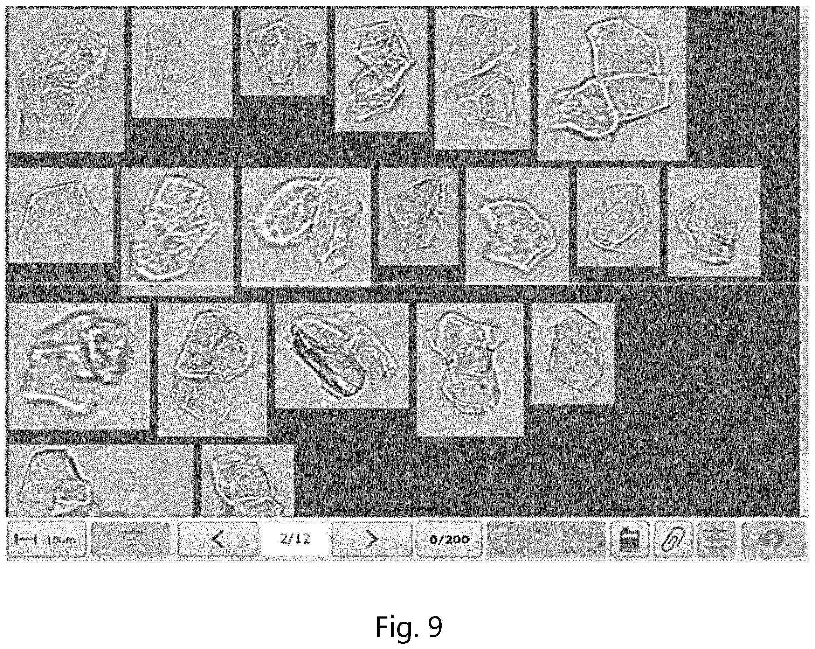

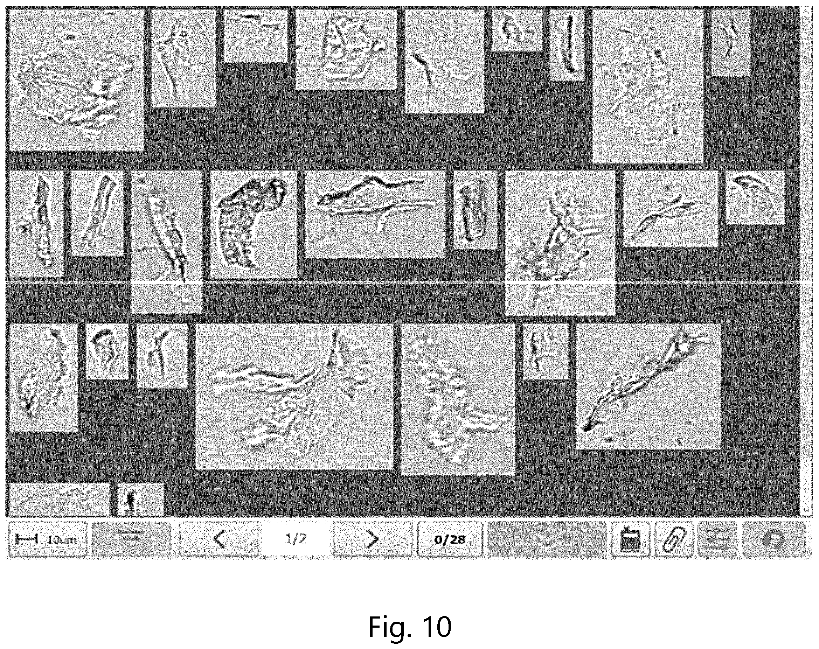

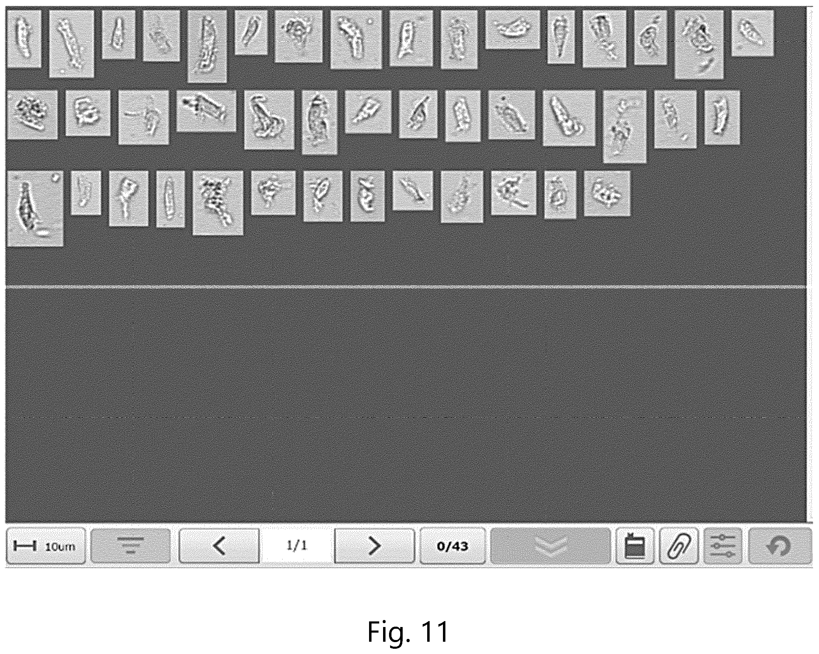

When the thus obtained immobilized squamous epithelial cells derived from each of the cervine, sheep, and porcine stomachs were analyzed by a urine sediment analyzer, AUTION EYE^{®} AI-4510 manufactured by Arkray, Inc., the cells were detected as SQEC. The images of the immobilized squamous epithelial cells derived from each of the cervine, sheep, and porcine stomachs, obtained by AUTION EYE^{®} AI-4510, are shown in FIGs. 9 to 11, respectively.

## Claims

1. Use of squamous epithelial cells derived from a mammal as a quality control substance in a measurement using a urine sediment analyzer;
wherein the squamous epithelial cells are mucosal epithelial cells derived from human oral cavity or surface mucous cells derived from bovine stomach, cervine stomach, sheep stomach, or porcine stomach.

2. The use according to claim 1, wherein the squamous epithelial cells are used as measurement controls for controlling the detection accuracy for detecting squamous epithelial cells in urine.

3. The use according to claim 1 or claim 2, wherein the squamous epithelial cells that are used as a quality control substance are mixed with an aqueous medium.

4. The use according to claim 3, wherein the aqueous medium has a buffering capacity.

5. The use according to any one of claims 1 to 4, wherein the squamous epithelial cells are immobilized.

6. The use according to any of claims 1 to 5, wherein the bovine stomach is bovine reticulum or bovine omasum.

7. A method for controlling the measurement accuracy of an analyzer that measures the cell number concentration of squamous epithelial cells in a sample, by counting the number of particles whose form is similar to the form of the squamous epithelial cells in the sample, the method comprising the steps of:
measuring the cell number concentration in a quality control substance comprising squamous epithelial cells derived from a mammal, using the analyzer, to obtain a measured value; and
determining the measurement accuracy of the analyzer by comparing the measured value with a reference concentration;
wherein the squamous epithelial cells derived from a mammal are mucosal epithelial cells derived from human oral cavity or surface mucous cells derived from bovine stomach, cervine stomach, sheep stomach, or porcine stomach.

8. The method for controlling the measurement accuracy according to claim 7, wherein the sample is urine.

9. The method for controlling the measurement accuracy according to claim 7 or claim 8, wherein the bovine stomach is bovine reticulum or bovine omasum.

## Patentansprüche

1. Verwendung von Plattenepithelzellen, die von einem Säugetier stammen, als eine Qualitätskontrollsubstanz in einer Messung unter Verwendung eines Urinsediment-Analysators;
wobei die Plattenepithelzellen Schleimhaut-Epithelzellen, die von einer menschlichen Mundhöhle stammen, oder Oberflächenschleimzellen, die von einem Rindermagen, Hirschmagen, Schafmagen oder Schweinemagen stammen, sind.

2. Verwendung nach Anspruch 1, wobei die Plattenepithelzellen als Messkontrollen zur Kontrolle der Nachweisgenauigkeit für das Nachweisen von Plattenepithelzellen in Urin verwendet werden.

3. Verwendung nach Anspruch 1 oder Anspruch 2, wobei die Plattenepithelzellen, die als eine Qualitätskontrollsubstanz verwendet werden, mit einem wässrigen Medium gemischt werden.

4. Verwendung nach Anspruch 3, wobei das wässrige Medium eine Pufferfähigkeit aufweist.

5. Verwendung nach einem der Ansprüche 1 bis 4, wobei die Plattenepithelzellen immobilisiert sind.

6. Verwendung nach einem der Ansprüche 1 bis 5, wobei der Rindermagen ein Rindernetzmagen oder ein Rinderblättermagen ist.

7. Verfahren zur Kontrolle der Messgenauigkeit eines Analysators, der die Zellanzahlkonzentration von Plattenepithelzellen in einer Probe misst, durch Zählen der Anzahl von Partikeln, deren Form der Form der Plattenepithelzellen in der Probe ähnlich ist, wobei das Verfahren die Schritte umfasst:
Messen der Zellanzahlkonzentration in einer Qualitätskontrollsubstanz, die Plattenepithelzellen umfasst, die von einem Säugetier stammen, unter Verwendung des Analysators, um einen Messwert zu erhalten; und
Bestimmen der Messgenauigkeit des Analysators durch Vergleichen des Messwerts mit einer Referenzkonzentration;
wobei die Plattenepithelzellen, die von einem Säugetier stammen, Schleimhaut-Epithelzellen, die von einer menschlichen Mundhöhle stammen, oder Oberflächenschleimzellen, die von einem Rindermagen, Hirschmagen, Schafmagen oder Schweinemagen stammen, sind.

8. Verfahren zur Kontrolle der Messgenauigkeit nach Anspruch 7, wobei die Probe Urin ist.

9. Verfahren zur Kontrolle der Messgenauigkeit nach Anspruch 7 oder Anspruch 8, wobei der Rindermagen ein Rindernetzmagen oder ein Rinderblättermagen ist.

## Revendications

1. Utilisation de cellules épithéliales squameuses dérivées d'un mammifère comme substance de contrôle de la qualité lors d'une mesure à l'aide d'un analyseur de sédiments urinaires ;
dans laquelle les cellules épithéliales squameuses sont des cellules épithéliales muqueuses dérivées d'une cavité buccale humaine ou des cellules muqueuses de surface dérivées d'un estomac de bovin, d'un estomac de cervin, d'un estomac de mouton, ou d'un estomac de porcin.

2. Utilisation selon la revendication 1, dans laquelle les cellules épithéliales squameuses sont utilisées comme des contrôles de mesure pour contrôler la précision de détection pour détecter des cellules épithéliales squameuses dans de l'urine.

3. Utilisation selon la revendication 1 ou la revendication 2, dans laquelle les cellules épithéliales squameuses qui sont utilisées comme substance de contrôle de la qualité sont mélangées avec un milieu aqueux.

4. Utilisation selon la revendication 3, dans laquelle le milieu aqueux a une capacité de tampon.

5. Utilisation selon l'une des revendications 1 à 4, dans laquelle les cellules épithéliales squameuses sont immobilisées.

6. Utilisation selon l'une quelconque des revendications 1 à 5, dans laquelle l'estomac de bovin est un réticulum de bovin ou un omasum de bovin.

7. Procédé destiné à contrôler la précision de mesure d'un analyseur qui mesure la concentration en nombre de cellules des cellules épithéliales squameuses dans un échantillon, en comptant le nombre de particules dont la forme est similaire à la forme des cellules épithéliales squameuses dans l'échantillon, le procédé comprenant les étapes suivantes :
la mesure de la concentration en nombre de cellules dans une substance de contrôle de la qualité comprenant des cellules épithéliales squameuses dérivées d'un mammifère, à l'aide de l'analyseur, pour obtenir une valeur mesurée ; et
la détermination de la précision de mesure de l'analyseur en comparant la valeur mesurée à une concentration de référence ;
dans lequel les cellules épithéliales squameuses dérivées d'un mammifère sont des cellules épithéliales muqueuses dérivées d'une cavité buccale humaine ou des cellules muqueuses de surface dérivées d'un estomac de bovin, d'un estomac de cervin, d'un estomac de mouton, ou d'un estomac de porcin.

8. Procédé destiné à contrôler la précision de mesure selon la revendication 7, dans lequel l'échantillon est de l'urine.

9. Procédé destiné à contrôler la précision de mesure selon la revendication 7 ou la revendication 8, dans lequel l'estomac de bovin est un réticulum de bovin ou un omasum de bovin.
